Europäisches Patentamt

European Patent Office

Office européen des brevets

(19)

(11) Veröffentlichungsnummer: **0 156 277**
A2

(12) **EUROPÄISCHE PATENTANMELDUNG**

(21) Anmeldenummer: 85103093.2

(22) Anmeldetag: 18.03.85

(51) Int. Cl.⁴: **A 61 K 31/325**
A 61 K 47/00

(30) Priorität: 29.03.84 DE 3411627

(43) Veröffentlichungstag der Anmeldung:
02.10.85 Patentblatt 85/40

(84) Benannte Vertragsstaaten:
AT BE CH DE FR GB IT LI NL

(71) Anmelder: BAYER AG
Konzernverwaltung RP Patentabteilung
D-5090 Leverkusen 1 Bayerwerk(DE)

(72) Erfinder: Voege, Herbert, Dr.
Martin-Buber-Strasse 41
D-5090 Leverkusen 3(DE)

(72) Erfinder: Rast, Hubert, Dr.
Hamberger Strasse 25
D-5090 Leverkusen 3(DE)

(54) Stabilisierte anthelmintische Formulierungen.

(57) Die vorliegende Erfindung betrifft stabilisierte anthelmintische Formulierungen von Febantel auf Basis von mit Wasser
mischbaren organischen Verdünnungsmitteln, die 1: 10 mit
Wasser verdünnt einen pH Wert von etwa 3 bis 5 besitzen.

EP 0 156 277 A2

Croydon Printing Company Ltd.

BAYER AKTIENGESELLSCHAFT    5090 Leverkusen, Bayerwerk

Konzernverwaltung RP
Patentabteilung    Rt/lic


Stabilisierte anthelmintische Formulierungen

Die vorliegende Erfindung betrifft stabilisierte anthelmintische Formulierungen von Febantel, ihre Herstellung und Verwendung.

Febantel, (N- {2-[2,3-Bis-(methoxycarbonyl)-guanidino]
5-(phenylthio)-phenyl} -2-methoxyacetamid) wird als Arzneimittel zur Bekämpfung von verschiedenen Würmern bei
Tieren verwendet. Es wird z. B. in Form von Suspensionen,
Granulaten, Pulvern oder Tabletten eingesetzt. Von besonderem Interesse sind aber Formulierungen, bei denen der
Wirkstoff in gelöster Form vorliegt und die entweder injiziert werden können oder als Lösungen auf die Haut aufgebracht werden. Letztere Formulierungen werden häufig als
pour-on oder spot-on Formulierungen bezeichnet.

Das Problem der gewünschten Lösungen liegt darin, daß
der Wirkstoff in gelöster Form chemisch wenig stabil ist.
Bei den Lösungsmitteln handelt es sich immer um organische Lösungsmittel mit oder ohne Wasseranteil, da der
Wirkstoff in Wasser allein praktisch unlöslich ist.

Es wurde nun gefunden, daß Formulierungen von Febantel
auf Basis von mit Wasser mischbaren organischen Verdünnungsmitteln stabilisiert werden können, indem man den

Le A 22 735

pH-Wert der 1 : 10 mit Wasser verdünnten Formulierung feststellt und die Formulierung mit der zur Einstellung eines pH-Werts von etwa 3 - 5 erforderlichen Menge einer Base, Säure oder Puffersubstanz versetzt.

Die Erfindung betrifft Formulierungen von Febantel, die nach diesem Verfahren stabilisiert worden sind sowie das Verfahren zu ihrer Herstellung.

Als Lösungsmittel für Febantel seien genannt organische, mit Wasser mischbare Lösungsmittel, z. B.

Etheralkohole wie Glycerinformal,
Heterocyclen wie N-Methyl-pyrrolidon,
Ester wie Dimethylsulfoxid oder Ethyllactat
Esteramide wie Dimethylacetamid.

Auch weniger wassermischbare Lösungsmittel wie Benzylalkohol, Benzylbenzoat oder Phenylaethanol können allein oder im Gemisch mit den vorgenannten Lösungsmitteln zum Einsatz kommen.

Der Wassergehalt der Lösungsmittel soll <3 % bevorzugt <1 % sein.

Die Konzentration des Wirkstoffs im Lösungsmittel beträgt 1 - 20 Gew.-%, bevorzugt 2 - 15 %, besonders bevorzugt 5 - 10 %.

Die Formulierung kann noch weitere übliche Formulierungshilfsstoffe wie Konservierungsmittel, oberflächenaktive

Le A 22 735

Stoffe, Spreitmittel, Geschmacksmittel, Farbstoffe, Stabilisatoren, enthalten.

Unter Spreitmitteln werden solche öligen Flüssigkeiten verstanden, die sich auf der Haut besonders
gut verteilen. Sie sind als solche in der Kosmetik
bekannt. Nach einem Vorschlag von R. Keymer, Pharm.
Ind. 32, 577 (1970) können sie z.B. durch ihre Oberflächenspannung gegen Luft charakterisiert werden, die
danach weniger als 30 dyn/cm betragen sollte. Diese
Spreitung kann auch nach dem sogenannten Abklatschtest
auf der menschlichen Haut experimentell bestimmt
werden (z.B. bei R. Keymer, Pharm.Ind. 32, 577 (1970),
oder F.Neuwald, K.E.Fetting und A.Szakall, Fette-
Seifen-Anstrichmittel 64, 465 (1962).

Als Spreitmittel kommen praktisch alle Substanzen in
Betracht, welche die oben angegebenen Eigenschaften
aufweisen. Insbesondere sind die folgenden Verbindungsklassen bzw. Verbindungen geeignet:

Silikonöle verschiedener Viskosität:

Fettsäureester, wie Ethylstearat, Di-n-butyl-adipat,
Laurinsäurehexalester, Dipropylen-glykolpelargonat,
Ester einer verzweigten Fettsäure mittlerer Kettenlänge mit gesättigten Fettalkoholen $C_{16}$-$C_{18}$, Isopropylmyristat, Isopropylpalmitat, Capryl/Caprinsäureester
von gesättigten Fettalkoholen der Kettenlänge $C_{12}$-$C_{18}$,
Isopropylstearat, Ölsäureoleylester, Ölsäuredecylester,
Ethyloleat, Milchsäureethylester, wachsartige Fettsäureester wie künstliche Entenbürzeldrüsenfett, Dibutyl-

Le A 22 735

- 4 -

phthalat, Adipinsäurediisopropylester, letzterem verwandte Estergemische u.a.

Triglyceride, wie Capryl/Caprinsäuretriglycerid, Triglyceridgemische mit Pflanzenfettsäuren der Kettenlänge $C_8-C_{12}$ oder anderen speziell ausgewählten natürlichen Fettsäuren, Partialglyceridgemische gesättigter oder ungesättigter eventuell auch hydroxylgruppenhaltige Fettsäuren, Monodiglyceride der $C_8/C_{10}$-Fettsäuren und andere.

Fettalkohole, wie Isotridecylalkohol, 2-Octyldodecanol, Cetylstearylalkohol, Oleylalkohol.

Fettsäuren, wie z.B. Ölsäure.

Besonders gut geeignete spreitende Öle sind die folgenden: Isopropylmyristat, Isopropylpalmitat, Capryl/Caprinsäureester von gesättigten Fettalkoholen der Kettenlänge $C_{12}-C_{18}$, wachsartige Fettsäureester wie künstliches Entenbürzeldrüsenfett und Silikonöle.

Als Konservierungsmittel seien genannt Benzylalkohol, Hydroxybenzoesäureester wie -methyl- oder -propylester, Benzoesäure, Chlorbutanol, Phenylethylalkohol.

Als weitere Hilfsstoffe sind geeignet:
Haftvermittler, z.B. Carboxmethylcellulose, Methylcellulose und andere Cellulose- und Stärke-Derivate, Polyacrylate, Alginate, gelatine, Gummiarabicum, Polyvinylpyrrolidon, Polyvinylalkohol, Copolymere aus Methylvinylether und Maleinsäureanhydrid, Polyethylenglykole, Paraffine, Öle, Wachse, hydriertes Rizinusöl.

Le A 22 735

Kolloidale Kieselsäure oder Gemische der aufgeführten Stoffe.

Ferner seien genannt oberflächenaktive Stoffe wie z. B.

1. anionaktive, wie Na-Laurylsulfat, Fettalkoholethersulfate, Mono/Dialkylpolyglykoletherorthophosphorsäureester-Monoethanolaminsalz,
2. kationaktive, wie Cetyltrimethylammoniumchlorid,
3. ampholytische, wie Di-Na-N-lauryl-:-iminodipropionat oder Lecithin,
4. nicht ionogene, z.B. polyoxethyliertes Ricinzsöl, polyoxethyliertes Sorbitan-Monooleat, Sorbitan-Monostearat, Ethylalkohol, Glycerinmonostearat, Polyoxyethylenstearat, Alkylphenolpolyglykolether.

Die Neutralisierung kann mit Basen erfolgen, die physiologisch verträgliche Salze bilden. Als Basen seien genannt: Ammoniak, Amine (primäre, sekundäre, tertiäre) wie Methylamin, Ethylamin, Ethanolamin, Diethanolamin, Triethanolamin, basische Aminosäuren wie Lysin, Cholin, Arginin, sowie 2-Amino-2-hydroxymethyl-1,3-propandiol, N-Methylglukamin, ferner mit anorganischen Basen wie NaOH oder KOH.

Die Neutralisierung kann mit Säuren erfolgen, die physiologisch verträgliche Salze bilden. Als Säuren seien genannt: Anorganische Säuren wie Halogenwasserstoffsäure z. B. Salzsäure, Schwefelsäure, Phosphorsäure,

Le A 22 735

ferner organische Säuren wie Essigsäure, Propionsäure, Zitronensäure, Milchsäure, Ascorbinsäure, Benzoesäure, Salizylsäure.

Die Neutralisation kann auch durch Zugabe von Puffersubstanzen erfolgen. Als solche seien genannt Salze der obengenannten Basen mit starken Säuren wie Halogenwasserstoffsäure, Schwefelsäure, Phosphorsäure, Essigsäure, Zitronensäure.

Bevorzugt seien genannt Natriumacetat sowie Citrate.

Zur Durchführung des erfindungsgemäßen Verfahrens wird zweckmäßigerweise in üblicher Weise die gewünschte Formulierung hergestellt. Ein Teil Formulierung wird dann mit 10 Teilen Wasser verdünnt und mit einer Glaselektrode der pH-Wert der Mischung gemessen. Dann wird zur ursprünglichen Formulierung so viel einer Säure, Base oder eines Puffersalzes zugegeben, daß eine ebenfalls 1 : 10 verdünnte wässrige Mischung unter denselben Bedingungen gemessen einen pH-Wert von etwa 3 bis 5 erreicht.

Es ist aber auch möglich, daß nach der vorbeschriebenen Methode zunächst das Lösungsmittel bzw. das Gemisch der Formulierungshilfsmittel auf den gewünschten pH-Wert eingestellt wird und danach die gewünschte Menge Wirkstoff zugegeben wird.

Die erfindungsgemäßen Formulierungen zeigen eine gute und breite Wirkung gegen folgende Nematoden und Cestoden:

1.  Hakenwürmer (z. B. Uncinaria stenocephala, Ancylostoma caninum, Bunostomum trigonocephalum)

Le A 22 735

2. Trichostrongyliden (z. B. Nippostrongylus muris, Haemonchus contortus, Trichostrongylus colubriformis, Ostertagia circumcinct

3. Strongyliden (z. B. Oesophagostomum columbianum)

4. Rhabditiden (z. B. Strongyloides ratti)

5. Spulwürmer (z. B. Ascaris suum, Toxocara canis, Toxascaris leonina),

6. Madenwürmer (z. B. Aspiculuris tetraptera),

7. Heterakiden (z. B. Heterakis spumosa),

8. Peitschenwürmer (z. B. Trichuris muris),

9. Filarien (z. B. Litomosoides carinii, Dipetalonema witei),

10. Cestoden (z. B. Hymenolepis nana, Taenia pisiformis, Echinococcus multilocularis)

Beispiel 1

5 g Febantel wurden in 100 ml Glycerinformal gelöst und die Lösung mit Essigsäure so eingestellt, daß bei einer Verdünnung von 1 Teil Formulierung in 10 Teilen Wasser sich ein pH-Wert von 4 einstellte. Von der so hergestellten Lösung wurde der Anfangsgehalt Febantel bestimmt und nach 3 Monaten Lagerung bei 35°C der Gehalt erneut bestimmt.

Nach 3 Monaten enthält die stabilisierte Lösung noch 4,3 % Febantel.

Eine unter denselben Bedingungen gelagerte unstabilisierte Lösung enthielt nur noch 0,7 % Febantel.

Beispiel 2

5,0 g Febantel
3,0 g Benzylalkohol
1,0 g Milchsäure
95,28 g n-Methylpyrrolidon
werden zusammengewogen und so lange gerührt, bis eine klare Lösung entstanden ist. 104,28 g Lösung entsprechen 100 ml. Bei einer Verdünnung 1 : 10 in Wasser stellte sich ein pH-Wert von 3,5 ein. Nach 3 Monaten Lagerung bei 35°C enthielt die Lösung noch 4,2 % Wirkstoff. Eine unter gleichen Bedingungen gelagerte nicht stabilisierte Lösung enthielt nach dieser Zeit nur noch 1 % Wirkstoff.

Le A 22 735

## Beispiel 3

10,0 g Febantel werden in 99,0 g Dimethylsulfoxid gelöst und ergeben dann 100 ml. Der Lösung werden 0,5 g Citronensäure und 0,2 g Trimethanolamin zugegeben und unter Rühren gelöst. Nach Verdünnen einen Probe mit Wasser im Verhältnis 1 : 10 stellt sich ein pH-Wert von 4,2 ein.

Nach 3 Monaten Lagerung bei 35°C weist dieses Muster noch einen Gehalt von 85 % des Anfangswertes auf, wogegen die nicht gepufferte Lösung nur noch einen Gehalt von 50 % des Anfangswertes zeigte.

Le A 22 735

Patentansprüche

1.  Stabilisierte Formulierungen von Febantel auf Basis von mit Wasser mischbaren organischen Verdünnungsmitteln, die 1 : 10 mit Wasser verdünnt einen pH-Wert von etwa 3 bis 5 besitzen.

2.  Formulierungen gemäß Anspruch 1, die 1 : 10 mit Wasser verdünnt einen pH-Wert von etwa 4 besitzen.

3.  Verfahren zur Stabilisierung von Formulierungen von Febantel auf Basis von mit Wasser mischbaren organischen Verdünnungsmitteln, dadurch gekennzeichnet, daß man den pH-Wert der 1 : 10 mit Wasser verdünnten Formulierungen durch Zusatz der erforderlichen Menge einer Säure, Base oder eines Puffersalzes auf einen Wert von etwa 3 bis 5 einstellt.

4.  Verwendung der stabilisierten Formulierungen gemäß Anspruch 1 als Anthelmintika.

5.  Verwendung von pour on Formulierungen von Febantel, die nach dem Verfahren gemäß Anspruch 3 stabilisiert worden sind, als Anthelmintika.

6.  Verwendung von Projektionslösungen von Febantel, die nach dem Verfahren gemäß Anspruch 3 stabilisiert werden, sind als Anthelmintika

Le A 22 735